# EUROPEAN PATENT APPLICATION

(11) **EP 4 151 270 A1**
(43) Date of publication of application: **22.03.2023**
(21) Application number: 21197593.3
(22) Date of filing: 20.09.2021
(51) Int. Cl.: A61N 1/02, A61N 1/372, G16H 20/40

(54) **SYSTEM FOR DATA COMMUNICATION AND RESPECTIVE METHOD**

(71) Applicant: BIOTRONIK SE & Co. KG, 12359 Berlin (DE)
(72) Inventor: DÖRR, Thomas, 12437 Berlin (DE)

(57) **Abstract**

The invention is directed to a system for data communication and a respective method in which provides at least near real-time control of at least one implantable medical device, wherein the system comprises the at least one medical device (110, 310, 320, 400), at least one relay device (120, 330) and a remote computer facility (150, 340), wherein to each of the at least one medical device one of the at least one relay device is assigned and located in proximity to this medical device, wherein each of the at least one medical device is configured to establish a bidirectional communication connection (A, B) to the remote computer facility via the assigned relay device and a communications network (125) for data exchange, wherein each of the at least one relay device comprises an interface (280) to the communications network, wherein the communications network uses the fifth generation technology standard for broadband cellular networks or a comparable standard or a higher standard (5G communications network) for said bidirectional communication connection.

## Description

The invention is directed to a system comprising at least one implantable medical, at least one relay device and a remote computer facility, wherein to each of the at least one medical device one of the at least one relay device is assigned and located in proximity to this medical device. The invention is further directed to an operation method of such system as well as a computer program product and a computer readable data carrier.

A implantable medical device is an active or passive implantable medical device, for example, an implant such as a pacemaker (with leads), a sensor implant like a heart rhythm monitor, an Implantable Leadless Pacer (ILP), an Implantable Leadless Pressure Sensor (ILPS), an Implantable Cardiac Defibrillator (ICD) or a non-transvenous ICD, a device that delivers spinal cord stimulation (SCS), deep brain stimulation (DBS) or neurostimulation, a drug pump or any other medical implant. Such medical device may deliver one or more therapeutic substances, e.g. a drug pump, contain sensors that collect physiological signals in order to monitor the health status of the patient and/or deliver a therapy to the patient, for example using electromagnetic waves.

The scope of operation or computing power of such implantable medical devices is limited as it is its energy resource and size. However, in some situations, a fast computing power support or data input from an external computer facility is needed in order to recognize dangerous situations with higher accuracy or to adapt the program provided inside the medical device to any new condition occurred with the patient.

Document US 2006/0293607 A1 discloses a system and method for communicating data and signals using a repeater or base station in the proximity to an implantable device such as an implantable heart failure monitor within a patient that achieves early detection of congestive heart failure (CHF). The known device alerts the patient and/or the attending physician when a diagnostic threshold is reached which is indicative of the progression of CHF. In the known system the division of sensing and analysis structures can be shared between implanted and external devices. A low-power radio transceiver operating in the MICS band is used for linking the device to a proximate base station. The base station communicates with one or more remote locations via telecommunications or wideband networks to permit monitoring patient data and programming the device remotely.

However, the above known system may not be sufficient to provide immediate action in a life-threatening situation or to provide complex calculations.

Accordingly, it is an object of the present invention to provide a system and method for medical data communication as well as computer program product and computer readable data carrier which provide near real-time external computing support under limited energy resources.

The above object is solved by a system having the features of claim 1, a method with the features defined in claim 11, a computer program product with the features of claim 14 and a computer readable data carrier with the features of claim 15.

In particular, the above problem is solved by a system for data communication comprising at least one implantable medical device, at least one relay device and a remote computer facility, wherein to each of the at least one medical device one of the at least one relay device is assigned and located in proximity to this medical device, wherein each of the at least one medical device is configured to establish a bidirectional communication connection to the remote computer facility via the assigned relay device and a communications network for data exchange in order to provide at least near real-time control of the at least one medical device, wherein each of the at least one relay device comprises an interface to the communications network, wherein the communications network uses the fifth generation technology standard for broadband cellular networks or a comparable standard or a higher standard (in short: 5G communications network) for said bidirectional communication connection.

The above system comprises the components
- at least one implantable medical device, for example several hundreds of implantable medical devices of the same type or of different types of medical device,
- at least one relay device located in proximity to the medical device to which it is assigned, wherein the system may comprise several hundreds of relay devices, and
- a remote computer facility.

The system further comprises a 5G communications network.

The at least one medical device may be any device as indicated above which is configured to be implanted partly or fully within a patient's body and is configured to monitor the health condition of a patient and/or to deliver a therapy to the patient. Particularly, the medical device may be, for example, any implant, a pacemaker, ILP, ICD, non-transvenous ICD, a sensor implant like a heart rhythm monitor, a device that delivers spinal cord stimulation (SCS), deep brain stimulation (DBS) or neurostimulation, a drug pump or any other medical implant. In one embodiment, the medical device is an active electronic implant for permanent implantation or for temporary implantation. The medical device may consist of one hermetically closed part but alternatively may consist of two or more parts (e.g. a defibrillator and an ILP). The two or more parts of the medical device may be located in different regions of the patient's body and, accordingly, may be implanted at different locations of the patient's body. The two or more parts of the medical device may communicate with each other. However, if the medical device has two or more parts, only one of these parts may comprise the communication module configured to communicate with the remote computer facility via the assigned relay device. This is advantageous with regard to the size and the power consumption of the medical device parts which do not communicate with the relay device.

The remote computer facility may comprise any hardware computer or virtual (cloud) computing facility which may include a single computer and network of computers. The remote computer facility may comprise at least one processor which may be operated centrally (on-premises) or de-centrally on multiple hardware and software instances (hybrid or cloud-based). The at least one processor of the remote computer facility performs substantial computations, including numerous arithmetic operations and logic operations without human intervention.

The at least one relay device (also referred to as relay station) may be a mobile device a permanently installed device such as a desktop computer, laptop computer, a notebook, tablet computer, a server computer, a mobile phone, a smartphone. The at least one relay device may be partially or fully extracorporeally to the patient's body. This means that it may be attached to or partly implanted to the patient's body but externally accessible, for example, in order to exchange a power source. The relay device may be a second medical device, different from the at least one implantable medical device defined above.

Each of the at least one relay device and of the at least one medical device may comprise at least one main processor, as well, that performs substantial computations, including numerous arithmetic operations and logic operations without human intervention.

As indicated above, one relay device is assigned to one of the at least one medical device and located in proximity to this medical device. This means that the assigned relay device and medical device form communication partners and are capable of establishing a communication connection for data exchange. In order to reduce latency in communication, the assigned relay device and medical device are located close to each other, for example no further than 100 m away, preferably no further than 30 m away, more preferably no further than 10 m away.

It is emphasized that each of the at least one relay device and the remote computer facility comprises an interface to the communications network that uses the fifth generation technology standard for broadband cellular networks (5G) or a comparable standard or a higher standard for said bidirectional communication connection with the remote computer facility. This means that such communication connection is a connection according to the International Mobile Telecommunications-2020 (IMT-2020 Standard) which are the requirements issued by the ITU Radiocommunication Sector (ITU-R) of the International Telecommunication Union (ITU) in 2015 for 5G networks, devices and services. It further comprises 5G NR (New Radio) communication according to new radio access technology (RAT) developed by 3GPP for the 5G (fifth generation) mobile network. The 5G NR communication was designed to be the global standard for the air interface of 5G networks. Comparable or higher standard means any similar or younger technological standard, in particular with regard to achievable latency, i.e. any standard with comparable or reduced latency with regard to the latency mentioned below.

This communication ensures an at least near real-time control of the at least one medical device, in particular, if, in one embodiment, the 5G communications network has a bandwidth of at least 100 Mbit/s and/or an air latency of 10 ms or less. Accordingly, the computing power of a remote computer facility can be used for real-time control of the at least one medical device, for example a beat-to-beat heart rhythm analysis (e.g. for tachyarrhythmia) and respective pacing control. This makes significantly more complex algorithms and principles accessible for the at least one medical device. The 5G network technology is characterized by a significantly higher data rate and, above all, significantly lower latency (about 1 ms). Likewise, the spatial availability of this network technology is improved significantly compared to the current network coverage.

The bidirectional communication connection of the at least one medical device to the remote computer facility comprises two sections. The first section is the bidirectional communication connection between the at least one medical device and the assigned relay device and the second section is the bidirectional communication connection between the assigned relay device and the remote computer facility. The second section is provided by the 5G communications network. The first section is described below in detail.

One application of the above-described inventive system may be a real-time morphological analysis of bodily signals of a patient determined by the at least one medical device, such as an electrocardiogram (ECG) signal, to back up therapy decisions of the medical device. For example, a subcutaneous defibrillator may send a relevant section of an ECG signal to the remote computer facility for extended assessment immediately before the shock is delivered via the aforementioned 5G data link with low latency, in order to seek a therapy decision from the remote computer facility. The remote computer facility is capable of providing an extended morphology assessment of the signal section and determines a therapy parameter from this assessment. This therapy parameter is then sent (again using the 5G data link with low latency) via the relay device to the medical device implanted in the patient's body. The shock delivery is then provided by the medical device (here comprising a defibrillation function.

In embodiment the remote computer facility performs at least one interactive test function based on a real-time data stream measured by the at least one medical device and transmitted to the remote computer facility. For example, a stimulation threshold test may be performed by having the medical device send the real-time electrogram to the cloud remote computer facility and controlling the stimulation threshold test in the cloud by sending the respective test amplitudes to the medical device and evaluating the stimulation response in the system beat-to-beat. If a stimulus is below the stimulus threshold, the system completes the evaluation within less than 50 ms, calculates and transmits a more reliable stimulation threshold to the medical device. Here, 5G technology is a prerequisite, as latency is typically higher than 50 ms for 4G communication, and 3 ms to 5 ms overall latency is expected for the system's communication comprising 5G communication between the at least one relay device and the remote computer facility. This embodiment may be applied to all clinical testing functions of the at least one medical device that rely on real-time assessment of bodily signals, in contrast to testing functions that have so far been performed by an on-site programmer.

In one embodiment, the maximum latency of the overall system, i.e. the latency measured from the sending of a request by the at least one medical device (i.e. by its communication module) and the receipt of the response from the remote computer facility by the at least one medical device (i.e. by it communication module) is less than 10 seconds, preferably less than 100 ms, more preferably less than 10 ms. This latency is determined by the latency of the 5G communication network, the communication latency of the medical device - relay device - communication and the latency within the relay device and the remote computer facility.

Each component of the system comprises a suitable communication module for bidirectional communication as indicated above. The communication between the at least one relay device and the remote computer facility is provided by the 5G communications network which may comprise additional relay stations. The communication between the medical device and the remote computer facility is mainly extracorporeally, with the medical device it may partly intracorporeally if the respective medical device is fully implanted within a patient's body. Each communication module comprises a sender, a receiver and/or a transceiver for sending and/or receiving the respective communication signals (also referred to as interface). Each communication module is electrically connected to the (main) processing unit of the respective component or may be integrated within the processing unit. For example, the communication module may comprise at least one processing unit separate from the main processing unit of the respective component. The communication module may be electrically connected to a data memory as well.

The communication between the at least one medical device and the assigned relay device may be provided by an interface realizing one communication technique wirelessly via the air using electromagnetic waves, for example, EDGE, EV-DO, Flash-OFDM, GPRS, HSPA, LoRaWAN, RTT, UMTS, Narrowband IoT, Bluetooth, WLAN (WiFi), ZigBee, NFC, LTE, Wireless USB, Wibree (BLE), MICS/MEDS and ULP-AMI, Ethernet or WiMAX in the radio frequency region, communication in the ISM band or IrDA or free-space optical communication (FSO) in the infrared or optical frequency region. Accordingly, the respective communication protocols, often with the same name (i.e. a system of rules that allows two or more entities of a communications system to transmit information via an kind of variation of a physical quantity, defining rules, syntax, semantics and synchronization of communication and possible error recovery methods and being implemented by hardware, software or a combination of both) or cooperating protocols (protocol suites) may be used, for example TCP/IP protocol suite (e.g. IPv6, TCP, UDP, SMTP, HTTP/2) also with TLS or SSL, IPX/SPX, X.25, AX.25, ebXML, Apple Talk, Bluetooth protocols (e.g. BR/EDR), Bluetooth 4.0 (BLE), ZigBee protocol, NFC protocol, IEEE 802.11 and 802.16 protocols, ETSI EN 301 389 (ULP-AMI, ULP-AMI-P) etc.

In one embodiment, the communication between the at least one medical device and the assigned relay device realizes a communication technique that uses low electrical current and/or low electrical energy consumption in the respective medical device, for example Bluetooth, Wibree (BLE), MICS/MEDS (MedRadio), ULP-AMI, communication in the ISM band, ZigBee, WLAN (WiFi), inductive communication.

The communication module of the at least one relay device is configured such that it has an interface to one of the above-mentioned communication techniques for bidirectional communication with the at least one medical device on the one hand and on the other hand it comprises an interface to the 5G communications network for bidirectional communication with the remote computer facility. Accordingly, the communication module of the at least one relay device may be a combined or two-part communication module capable of communicating using both communication techniques. Accordingly, the communication module may comprise two different transceivers (one for each communication technique). Accordingly, the communication module of each component of the system comprises the respective hardware and software adapted to the above-mentioned communication techniques / communication protocols which are provided by the respective communication module. Further, it is necessary that the at least one medical device, at least one relay device and the remote computer facility comprise communication modules providing at least partly similar communication modules in order to "talk" to each other, i.e. to establish and maintain a communication connection between each other in order to transmit data, for example data of a bodily parameter of the patient and/or data of the medical device implanted within or attached to the patient's body. Sometimes the communication techniques are downward compatible which means that a communication module with a higher version of a communication protocol "understands" a communication module with a lower versions of a communication protocol, i.e. a working communication connection can be established by communication modules comprising different versions of the same communication module. Such situations shall be covered by the present invention.

Each component of the system may comprise the data memory which may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device.

In one embodiment, wherein real-time control of the at least one medical device comprises initiating at least one of a group of actions provided by the at least one medical device, wherein the group of actions comprises release of therapy, suppression of therapy delivery, adaption of a therapy modus, adaption of at least one therapy parameter, operation control of the at least one medical device, assessment and/or analysis of at least one measured bodily signal, checking of an action selected by the respective medical device, interactive operation test based on real-time data stream from the medical device. This is explained in above embodiments.

In one embodiment, the at least one medical device is configured such that the bidirectional communication connection to the remote computer facility is established only if required, e.g. one pre-defined condition is true, for example, if an action selected by the medical device needs to be checked or if the medical device needs input from the remote computer facility in order to provide the action. In this case, the energy used for communication is needed in the mentioned cases, only. For example, 5G-communications network and remote computer facility based assessment is used to safeguard therapy decisions (e.g. defibrillation shock delivery) for relatively infrequent therapy deliveries or "when in doubt."

In one embodiment, the at least one relay device and the remote computer facility are configured such that the bidirectional communication connection between the at least one relay device and the remote computer facility via the 5G communications network is permanently active. This decreases latency as the 5G communications connection does not need to set up.

In one embodiment, the at least one relay device is configured such that it, e.g. its communication module, cyclically performs a channel scan for the bidirectional communication connection with the assigned medical device and/or with the remote computer facility in order to seek for the channel with the best communication connection.

In one embodiment, the data exchanged by the bidirectional communication connection between the at least one medical device and the remote computer facility is encrypted by the respective medical device and/or the remote computer facility prior transmission using state-of-the-art encryption protocols and authentication methods to ensure data security.

In one embodiment, the communication module and/or a therapy delivery module and/or a therapy signal processing module and/or a bodily signal measurement module of each of the at least one medical device has a direct memory access (DMA) functionality in order to provide the saved data faster thereby decreasing overall latency. This means that an extremely fast and low-current data exchange takes place, which significantly decreases the latency within the at least one medical device.

In one embodiment, the at least one medical device is configured such that a pre-defined energy budget for bidirectional communication with the assigned relay device within a pre-defined time interval, e.g. one day, is not exceeded. This avoids large energy consumption by communicating data.

With regard to the above embodiment, since communication of data is dependent on the frequency of occurrence of, e.g. ECG, signal disturbances, the energy budget of the at least one medical device available for data transmission is managed by the main processor in such a way that no excessive discharge of the power source takes place. For this purpose, for example, a maximum energy budget for a predefined time period (e.g., daily) may be defined and if within the time period the budget is depleted, data transmission is stopped until the next time period. However, if the energy budget is not used up, it is credited to an energy account provided within the main processor of the at least one medical device that may be used up additionally in a subsequent time period. This embodiment has the advantage that the maximum energy consumption for data transmission is not exceeded in the device runtime and at the same time it is possible to use data transmission for a longer time in phases of greater interference.

In one embodiment, the at least one medical device is a non-transvenous defibrillator. The non-transvenous defibrillator is configured to detect a cardiac arrhythmia requiring therapy. Since sensing electrodes of such defibrillator are not directly connected to the heart, the signals used for cardiac rhythm assessment are often overlaid by interfering signals, such as muscle potentials, motion artifacts of the sensing electrodes, and/or external disturbances. State-of-the-art perception algorithms usually conditionally detect such disturbances and mask out the disturbed signal sections for cardiac rhythm analysis. However, the disadvantage of this suppression is a delay in the detection of life-threatening arrhythmias and an associated delay in the delivery of shock therapy. If the inventive system is used with the non-transvenous defibrillator as a medical device, the defibrillator comprises a separate communication module, which is preferably implemented in hardware, in order to be able to establish a low-current and latency-optimized connection with the at least one assigned relay device. The defibrillator with its communication module is configured to send at least a section of the measured ECG signal to the remote computer facility whenever an interference signal overlay of the signal has been detected. This means that the ECG signal is initially evaluated by the defibrillator's main processor without any external communication. However, if an interference signal overlay is detected by the main processor, the communication module is activated and the respective ECG signal section is transmitted from the data memory, to which the communication module has direct read access, to the relay device with a short time delay. In principle, all low-current communication methods currently used in medical devices (e.g. MICS band; Bluetooth Low Energy; Zigbee; ISM band) can be used for transmission between the internal communication module of the medical device (non-transvenous defibrillator) and the communication module of the assigned at least one relay device. The amount of data to be transmitted is small (e.g. for an ECG signal 1 to 2 ECG channels with a sampling rate of 512 Hz or 1024 Hz and a 8 to 12 bit amplitude resolution) compared with other medical devices and is transmitted to the assigned relay device with the mentioned methods without significant delay (a few ms).

The relay device forwards this ECG signal via a low-latency mobile 5G communications network to the remote computer facility, which is designed using complex analysis algorithms to analyze the transmitted ECG signal in real time despite superimposed interference and to send, for example a classification result of the ECG signal back to the implant via the assigned relay device. To ensure low-delay data processing at all times, the at least one relay device remains permanently connected to the 5G communications network and thereby connected to the remote computer facility. The server-based algorithms of the remote computer facility, in contrast to algorithms implemented with the very limited computing power in the medical device, are capable to perform complex rhythm evaluation in the presence of disturbed input signals with much more powerful signal analysis.

The externally determined classification result is made available to the main processor of the defibrillator and its classification result is supplemented accordingly (e.g. by increasing or decreasing detection counters). The classification result always also contains the authentication information and the time stamp of the associated ECG signal section, so that the correct assignment to the ECG signal section measured by the defibrillator can be made and the latency for the overall data transmission in the system is determined (i.e. from the defibrillator to the remote computer facility, the signal analysis there and the transmission of the classification result back to the defibrillator). If the overall latency determined in this way exceeds a pre-defined limit value, the external classification result may be discarded and, if the limit value continues to be exceeded, the then useless data transmission is stopped for a pre-define time interval so as not to waste unnecessary energy on data transmission.

As indicated above, in this embodiment, in order to provide a unique assignment of the externally determined signal classification, the sent signal section to be classified is automatically provided with a time stamp and authentication information (e.g. a specific label that assigns the signal to the specific defibrillator) generated by the internal communication module. In addition, end-to-end encryption of the ECG signals may be performed by the internal communication module of the medical device (defibrillator) or by a comparable additional cybersecurity layer over the 5G protocol by the assigned relay device.

In another embodiment, the system remote computer facility provides an active control of the timing of anti-tachycardia pacing (ATP). For example, the at least one medical device in the form of a leadless pacemaker may be capable of delivering an ATP, wherein the ATP timing is provided by the complex algorithms of the remote computer facility in response to ECG changes from pulse-to-pulse.

In another embodiment of the system, the remote computer facility may deliver a backup therapy in case of electrode failures in stimulating medical devices. Similarly, complex therapy optimization for a neurostimulator forming the medical device may be implemented using a closed loop approach for cyclic therapy optimization.

Generally, in one embodiment the system may realize all interactive medical device tests requiring real-time telemetry by means of a virtual programming algorithm provided by the cloud remote computer facility of the remote computer facility.

From the above explanation, also with regard to some embodiments, it becomes clear that the inventive system enables completely new, more complex ways of controlling, testing and evaluating medical devices, especially active implants. The usage of 5G communications network represents a significant differentiator in the field of implantable medical devices.

The above object is also solved by a method for data communication within a system comprising at least one implantable medical device, at least one relay device and a remote computer facility, wherein to each of the at least one medical device one of the at least one relay device is assigned and located in proximity to this medical device, wherein for data exchange of the at least one medical device with the remote computer facility for external analysis of the transferred data, the at least one medical device establishes a bidirectional communication connection to the remote computer facility via the assigned relay device and a communications network, wherein the at least one relay device establishes the bidirectional communication connection to the remote computer facility via the communications network, wherein the communications network uses the fifth generation technology standard for broadband cellular networks (5G) or a comparable standard or a higher standard for said bidirectional communication connection.

In one embodiment of the above method, the communication connection of the at least one medical device and the assigned relay device is established by a communication technique that uses low electrical current and/or low electrical energy consumption in the respective medical device, for example, Bluetooth, Wibree (BLE), MICS/MEDS, ULP-AMI, Zigbee, WLAN, communication in the ISM band, inductive communication.

In one embodiment of the above method, the at least one medical device establishes the bidirectional communication connection to the remote computer facility only if required, for example, if an action of the at least one medical device needs to be checked or if the at least one medical device needs input from the remote computer facility in order to provide the action.

The above embodiments of the operation method have the same advantages as the above system. Embodiments of the system indicated above may be realized as the respective operation method analogously. It is referred to the above explanation of the system in this regard.

The above method may, further, be realized as a computer program which comprises instructions which, when executed, cause the system to perform the steps of the above method which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, the above defined method may be part of a computer program product comprising instructions which, when executed by a processor, cause the processor to perform the steps of the above defined method. Accordingly, a computer readable data carrier storing such computer program product is disclosed.

The present invention will now be described in further detail with reference to the accompanying schematic drawings, wherein
- Fig. 1: shows a first embodiment of the inventive system;
- Fig. 2: depicts a medical device and a relay device of the embodiment of the inventive system shown in Fig. 1 in more detail;
- Fig. 3: shows a second embodiment of the inventive system; and
- Fig. 4: depicts an embodiment of a medical device of a third embodiment of the inventive system.

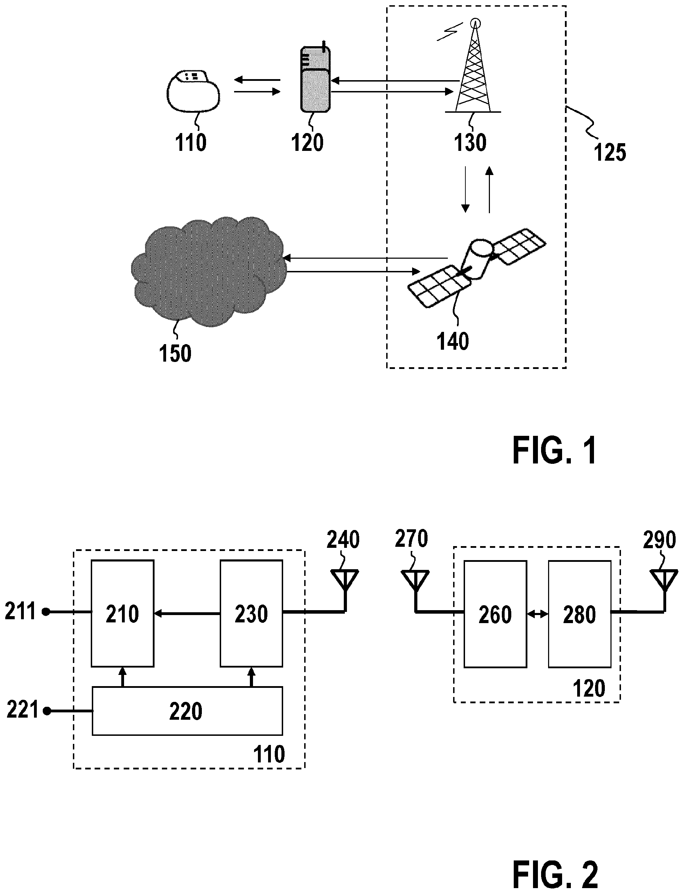

Fig. 1 shows a first embodiment of the inventive system comprising at least one medical device 110, for example a pacemaker or defibrillator, at least one relay device 120, e.g. a smartphone, and a remote computer facility 150. It is referred to the above explanation regarding the construction and properties of the remote computer facility, which may be cloud-based. Within such system hundreds of medical devices 110 and relay devices 120 may be organized. In order to ease explanation, it is referred to only one medical device and relay device in this and the following embodiments, but the invention is not limited by this number. The medical device 110 implanted within a patient transmits sensor data that the medical device 110 has recorded to the patient's own relay device 120 (i.e. the relay device 120 assigned to the patient and thereby to the medical device 110 of this patient, wherein the relay device 120 is located within several meters in the vicinity of the medical device 110). The sensor data are then transmitted from the relay device 120 via a 5G communications network 125 to the remote computer facility 150, wherein the 5G communications network 125, for example, comprises a base station 130 and a satellite 140. The remote computer facility 150 is a cloud-based real-time evaluation system. The initial transmission path from the medical device 110 to the assigned relay device 120 is required for energy reasons, as the power supply and transceiver arrangement (body attenuation) of the medical device 110 is not designed for direct communication with a 5G communications network. In the remote computer facility 150 the sensor data are evaluated and control signals/parameter determined on basis of these sensor data are transmitted back to the medical device 110 in real time via the aforementioned communication path, so that a medical device function, such as therapy delivery or therapy adjustment, can be performed directly in the medical device 110 just after receipt of these control signals/parameter.

The advantage of the remote programming of the medical device 110 using the above system comprising the 5G communications network is that the control function by the remote computer facility 150 is provided in near real time and thus essential medical device algorithms for medical device control can be relocated to the cloud-based evaluation by the remote computer facility 150. In order to realize such a control of a function suitable for the medical device function, in addition to the low latency of the 5G communications network (typically 1 ms), a short latency for the bidirectional data transmission between the medical device 110 and the relay device 120 is provided, so that the total response time to a control request of the medical device 110 of the remote computer facility 150 is sufficiently fast. Accordingly, in this embodiment a bidirectional telemetry using an existing bidirectional MICS band telemetry communication module (interface) with a data rate of 400 kbit/s is used for data transmission between the medical device 110 and the relay device 120. Alternatively, a Bluetooth Low Energy connection (or similar) between the medical device 110 and the relay device 120 can be used.

For example, a heart rhythm analysis is performed using a beat-to-beat analysis using the system as indicated above and shown in Fig. 1. Here, the analysis response takes place by the next expected heartbeat, i.e. is completed within 200 ms for a tachyarrhythmia analysis.

Fig. 2 shows a block diagram of the medical device 110 and the relay device 120. The medical device 110 includes a therapy control and delivery unit 210 (e.g., defibrillation unit of a subcutaneous defibrillator), an electrode 211 connected with the therapy control and delivery unit 210, a sensor data derivation and analysis unit 220 (e.g., for a subcutaneous ECG), an electrode 221 connected with the sensor data derivation and analysis unit 220, and a communication module 230 with a transceiver 240. Here, the communication module 230 includes, for example, a hardware-based streaming unit to enable low-delay transmission of the sensor data without adversely affecting therapy control or other medical device functions. Alternatively, a BLE SoC (system on chip) can be used here, which has one or more dedicated low-power processors and can thus fulfill the real-time requirement independently of the therapy control processor. Telemetry to the relay device 120 is carried out in the MICS band or using the BLE protocol, for example. The relay device 120 comprises a two-part communication module and has a communication connection to the medical device 110 via a corresponding first part of a first communication module part 260 comprising a transceiver 270, wherein the first part of the communication module 260 with its transceiver 270 is capable of receiving the data sent from the transceiver 240 of the medical device 110. The relay device 120 further comprises a 5G mobile radio second communication module part 280 having an adapted transceiver 290 for communication over the 5G communications network 125 to the remote computer facility 150.

The sensor signals from the sensor data derivation and analysis unit 220 of the medical device 110 are thus transmitted with a short delay (latency) to the relay device 120, forwarded by the latter to the cloud remote computer facility 150, are processed there and the results of the data processing are transmitted back to the medical device 110 along the similar communication path.

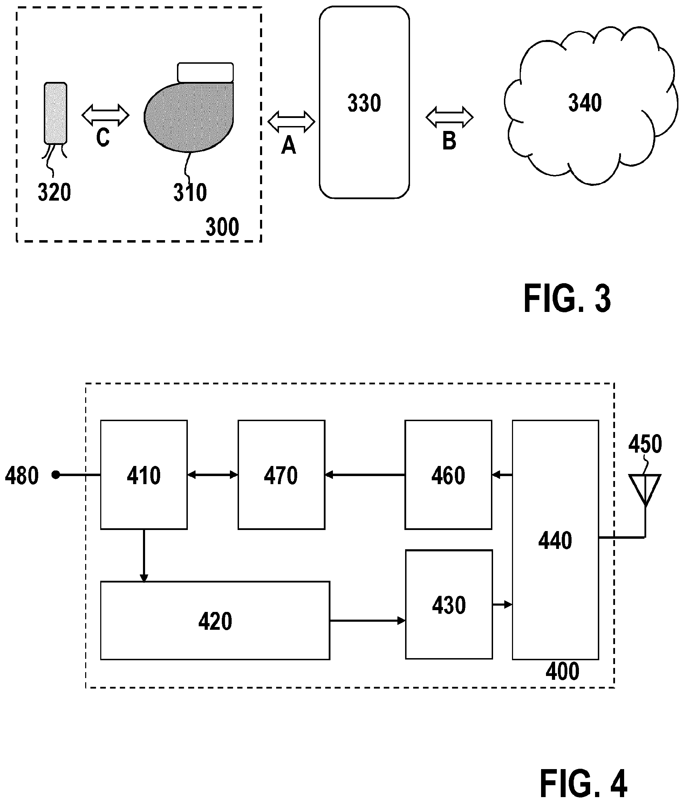

Fig. 3 shows another embodiment for the described communication system. Here, the at least one medical device consists of one or more separate parts, for example a first medical device part 310 and optionally one second medical device part 320, wherein the at least one medical device (i.e. its two parts) is implanted into the patient's body 300. For example, the first medical device part 310 is a non-transvenous defibrillator and the second medical device part 320 is a leadless pacemaker (ILP).

According to the invention, the medical device is connected to by a communication module of the first medical device part 310 to an external relay device 330 using a connection A which is operated with a very low power telemetry protocol suitable for medical device, such as Bluetooth Low Energy, MICS band, Zigbee, ISM band, ULP-AMI. This communication connection using one of the above-mentioned well-known telemetry protocols is controlled by the communication module of the first medical device part 310 with appropriate hardware, so that it can also support the latent time requirements, in principle.

The bidirectional communication connection A of the medical device to the relay device 330 is controlled by the first medical device part 310 as indicated above and is activated on the first medical device part's side only if required. However, a communication module for the communication connection A in the relay device 330 is permanently active, so that communication between the relay device 330 and the first medical device part 310 can be started on demand without significant delay. Thus, depending on the communication protocol, the relay device 330 may cyclically perform a channel scan to activate communication with the first medical device part 310 in an appropriate channel at any time. The relay device 330 further comprises a communication module suitable for a 5G communications network that establishes a bidirectional low-latency communication connection B to a cloud remote computer facility 340 if required by the first medical device part. In order to communicate data without any time delay as well, the latter communication connection B remains permanently established.

A further data link C may be established between the first medical device part 310 and the second medical device part 320, if required. In this case, the first medical device part 310 acts as a communication hub in the patient's body and may thus also connect one or more further medical device parts to the relay device 330. For example, as shown in Fig. 3, the second medical device part 320 (e.g. ILP) may be capable of delivering an ATP and the first medical device part 310 (e.g. non-transvenous defibrillator) may send ECG signals determined by the first medical device part 310 to the cloud remote computer facility 340 in real time to adjust the timing of ATP delivery by the second medical device part 320 in response to ECG changes from pulse-to-pulse of the patient's heart.

Fig. 4 shows a block diagram of an embodiment of one medical device 400 providing communication via the 5G communications network as indicated in Fig. 1 and 3 above. The medical device 400 comprises a perception and therapy unit 410 connected to an electrode or sensor line 480 to continuously sense bodily signals from the patient's body and to deliver therapy when needed. The perception and therapy unit 410 is connected on the one hand to a detection and therapy control unit 470 and on the other hand to a hardware ring buffer 420, which temporarily stores the recorded, digitized bodily signals. The detection and therapy control unit 470 initially decides continuously and autonomously on a possible need for therapy and, if necessary, controls the therapy output of the perception and therapy unit. In the event of a difficult therapy decision or complex therapy control (see above examples), the detection and therapy control unit 470 initiates latency-optimized 5G communication to support the therapy decision or control. For this purpose, the bodily signals derived from the hardware ring buffer 420 are first continuously encrypted in the hardware communication controller by means of a so-called streaming unit 430 via hardware and provided with authentication information. For authentication, e.g., Elliptic Curve Cryptography and an 128 bit AES (Advanced Encryption Standard) for encryption are provided by hardware. A communication module 440 (medical low power radio) with its transceiver 450 is then used to transmit the encrypted data stream with low latency (e.g. in the MICS band) to the external relay device for further communication with a 5G communications network. The latency for communication between the communication module 440 and the relay device is caused mainly by the block size for the AES encryption (128 bit). To reduce the latency for the encrypted signals, smaller signal blocks may be produced and the remaining bytes up to 128 bit block size are filled with placeholders. This makes it possible to achieve an optimum latency with regard to the available bandwidth on the telemetry link and the data size/rate of the signals.

Along the bidirectional communication connection with the relay device, the medical device 400 receives in parallel the information from the cloud remote computer facility via transceiver 450 and MICS band communication module 440. In this case, no streaming formats are necessary, the data packets are authenticated and decrypted in a packet-oriented hardware unit 460 and their information is then forwarded to the detection and therapy control unit 470. All communication and function units/modules of the medical device 400 are connected with so-called DMA units (Direct Memory Access), so that an extremely fast and low-current data exchange is provided, which further decreases latency. In one embodiment, the similar hardware as in the medical device 400 is used in the relay device as the communication interface to the medical device, e.g. a communication module and transceiver similar to the communication module 440 and transceiver 450 of the medical device 400.

## Claims

1. System for data communication comprising at least one implantable medical device (110, 310, 320, 400), at least one relay device (120, 330) and a remote computer facility (150, 340), wherein to each of the at least one medical device one of the at least one relay device is assigned and located in proximity to this medical device, wherein each of the at least one medical device is configured to establish a bidirectional communication connection (A, B) to the remote computer facility via the assigned relay device and a communications network (125) for data exchange, wherein each of the at least one relay device comprises an interface (280) to the communications network, wherein the communications network uses the fifth generation technology standard for broadband cellular networks or a comparable standard or a higher standard (5G communications network) for said bidirectional communication connection.

2. The system of claim 1, wherein the 5G communications network has a bandwidth of at least 100 Mbit/s and/or an air latency of 10 ms or less.

3. The system of any of the previous claims, wherein the communication connection (A) of each of the at least one medical device (110, 310, 320, 400) and the assigned relay device (120, 330) realizes a communication technique that uses low electrical current and/or low electrical energy consumption in the respective medical device, for example, Bluetooth, Wibree (BLE), MICS/MEDS, ULP-AMI, Zigbee, WLAN, communication in the ISM band, inductive communication.

4. The system of any of the previous claims, wherein real-time control of the at least one medical device (110, 310, 320, 400) comprises initiating at least one of a group of actions provided by the at least one medical device comprising release of therapy, suppression of therapy delivery, adaption of a therapy modus, adaption of at least one therapy parameter, operation control of the at least one medical device, assessment and/or analysis of at least one measured bodily signal, interactive operation test based on real-time data stream from the medical device.

5. The system of any of the previous claims, wherein the at least one medical device (110, 310, 320, 400) is configured such that the bidirectional communication connection (A, B) to the remote computer facility (150, 340) is established only if required, for example, if an action needs to be checked or if the medical device needs input from the remote computer facility in order to provide the action.

6. The system of any of the previous claims, wherein the at least one relay device (120, 330) and the remote computer facility (150, 340) are configured such that the bidirectional communication connection (B) between the at least one relay device and the remote computer facility is permanently active.

7. The system of any of the previous claims, wherein the at least one relay device (120, 330) is configured such that it cyclically performs a channel scan for the bidirectional communication connection (A, B) with the assigned medical device (110, 310, 320, 400) and/or with the remote computer facility (150, 340).

8. The system of any of the previous claims, wherein the data exchanged by the bidirectional communication connection (A, B) between the at least one medical device (110, 310, 320, 400) and the remote computer facility (150, 340) is encrypted by the respective medical device and/or the remote computer facility prior transmission.

9. The system of any of the previous claims, wherein a communication module and/or a therapy delivery module and/or a therapy signal processing module and/or a bodily signal measurement module of each of the at least one medical device (110, 310, 320, 400) have a direct memory access (DMA) functionality.

10. The system of any of the previous claims, wherein the at least one medical device (110, 310, 320, 400) is configured such that a pre-defined energy budget for bidirectional communication with the assigned relay device (120, 330) within a pre-defined time interval, e.g. one day, is not exceeded.

11. Method for data communication within a system comprising at least one implantable medical device (110, 310, 320, 400), at least one relay device (120, 330) and a remote computer facility (150, 340), wherein to each of the at least one medical device one of the at least one relay device is assigned and located in proximity to this medical device, wherein for data exchange of the at least one medical device with the remote computer facility for external analysis of the transferred data, the at least one medical device establishes a bidirectional communication connection (A, B) to the remote computer facility via the assigned relay device and a communications network (125), wherein the at least one relay device establishes the bidirectional communication connection (B) to the remote computer facility via the communications network, wherein the communications network uses the fifth generation technology standard for broadband cellular networks (5G) or a comparable standard or a higher standard for said bidirectional communication connection.

12. The method of claim 11, wherein the communication connection (A) of the at least one medical device (110, 310, 320, 400) and the assigned relay device (120, 330) is established by a communication technique that uses low electrical current and/or low electrical energy consumption in the respective medical device, for example, Bluetooth, Wibree (BLE), MICS/MEDS, ULP-AMI, Zigbee, WLAN, communication in the ISM band, inductive communication.

13. The method of any of the claims 11 to 12, wherein the at least one medical device (110, 310, 320, 400) establishes the bidirectional communication connection (A, B) to the remote computer facility (150, 340) only if required, for example, if an action of the at least one medical device needs to be checked or if the at least one medical device needs input from the remote computer facility in order to provide the action.

14. A computer program product comprising instructions which, when executed by a processor, cause the processor to perform the steps of the method according to any of the claims 11 to 13.

15. Computer readable data carrier storing a computer program product according to claim 14.
